# DEMANDE DE BREVET EUROPEEN

(11) **EP 1 016 713 A1**
(43) Date de publication de la demande: **05.07.2000**
(21) Numéro de dépôt: 99403286.0
(22) Date de dépôt: 27.12.1999
(51) Int. Cl.: C12N 11/04, C12P 19/22

(54) **Alpha-amylase maltogénique immobilisee et son utilisation dans la fabrication d'un sirop riche en maltose**

(30) Priorité: 29.12.1998 FR 9816537
(71) Demandeur: Roquette Frères, 62136 Lestrem (FR)
(72) Inventeur: Duflot, Pierrick, 62136 Lacouture (FR); Fouache, Catherine, 62113 Sailly Labourse (FR)
(74) Mandataire: Touati, Catherine

(57) **Abrégé**

L'invention concerne une α-amylase maltogénique immobilisée, caractérisée par le fait qu'elle est adsorbée sur des particules d'au moins un support choisi dans le groupe constitué par les résines phénoliques, les résines acryliques et les résines polystyréniques.

Elle concerne également l'utilisation d'une telle α-amylase pour la fabrication d'un sirop riche en maltose.

## Description

L'invention concerne une α-amylase maltogénique immobilisée et son procédé de fabrication. Elle concerne encore un procédé de fabrication d'un sirop riche en maltose. Elle concerne aussi un procédé de fabrication d'un sirop riche en maltitol à partir d'un sirop riche en maltose obtenu par le procédé conforme à la présente invention. Elle concerne également un procédé de fabrication de maltitol cristallisé à partir d'un sirop riche en maltose obtenu par le procédé conforme à la présente invention.

On connaît déjà des procédés permettant d'obtenir des sirops riches en maltose. Parmi ces procédés, on peut notamment citer celui décrit par HODGE et Coll. dans "Cereal Chemistry" n° 25, pages 19-30, Janvier 1948, et qui comprend une étape de précipitation des dextrines limites par des solutions alcooliques et celui décrit par WOLFROM et THOMPSON dans "Methods in carbohydrate chemistry", 1962, pages 334-335.

D'autres procédés de fabrication de sirops riches en maltose ont encore été proposés comprenant une étape d'adsorption sur charbon des dextrines (US-A-4.194.623), une étape de chromatographie sur zéolithes ou résines cationiques ou anioniques (FR-A-2.510.581), une étape d'ultrafiltration des sirops de maltose (US-A-4.429.122), l'utilisation combinée de plusieurs enzymes différentes, à savoir une α-amylase, une β-amylase et une iso amylase ou une pullulanase (FR-A-2.012.831).

Cette dernière technique présente, par rapport aux précédentes, de nombreux avantages. Elle souffre néanmoins de certains inconvénients, dont notamment celui résidant dans le fait que les saccharifications doivent être effectuées à des teneurs en matières sèches très basses, de l'ordre de 20 g/l, pour obtenir une efficacité d'hydrolyse maximum des enzymes.

Le document FR-A-2.000.580 décrit un procédé de préparation d'un sirop à teneur élevée en maltitol par hydrogénation d'un sirop à teneur élevée en maltose qui est obtenu par liquéfaction d'un lait d'amidon à faible teneur en matières sèches jusqu'à un dextrose-équivalent inférieur à 2, le produit ainsi obtenu étant saccharifié sous l'action d'enzymes spécifiques.

Ce procédé est coûteux, d'un rendement médiocre et donne lieu à des problèmes de contamination bactérienne et à des phénomènes de rétrogradation de l'amylose. En outre, le sirop obtenu contient des proportions de polymères de degré de polymérisation (DP, dans la suite de la description) supérieur ou égal à 4, qui sont gênantes.

Plus récemment, le document US-A-5.141.859 a proposé un procédé de fabrication d'un sirop à teneur élevée en maltose, mettant en oeuvre deux étapes successives de saccharification. Ce document préconise en effet un procédé comprenant une première étape de saccharification en présence d'une β-amylase et une étape subséquente de saccharification en présence d'une α-amylase maltogénique. Selon ce document, l'α-amylase maltogénique est utilisée, après la première étape de saccharification à la β-amylase, pour hydrolyser les oligosaccharides (de DP3 à DP7) et essentiellement le maltotriose (trisaccharide), en maltose et glucose.

De manière surprenante et inattendue, la Société Demanderesse a constaté que des sirops, à teneur aussi élevée en maltose que ceux décrits dans le document US-A-5.141.859, pouvaient être obtenus en saccharifiant un lait d'amidon liquéfié au moyen d'une α-amylase maltogénique immobilisée.

A la connaissance de la Société Demanderesse, seul le document FR-A-2.356.665 a proposé d'immobiliser une α-amylase dite maltogénique, mais sur un support très particulier constitué de granules de caséine essentiellement revêtus d'une couche protéique perméable aux liquides dans laquelle l'enzyme est réticulée en commun avec de l'albumine d'oeuf par l'aldéhyde glutarique. En outre, selon ce document, l'utilisation (qui n'a pas été exemplifiée) d'α-amylase maltogénique immobilisée sur un support poreux pour le traitement d'un hydrolysat d'amidon s'avère théoriquement impossible pour des raisons d'empêchement stérique, la diffusion des oligosaccharides de DP6 à DP10 contenus dans un tel hydrolysat vers l'enzyme enfermée au sein de la structure poreuse étant empêchée compte tenu de la taille des molécules.

Contre toute attente et contrairement à l'enseignement du document FR-A-2.356.665, la Société Demanderesse a mis en évidence d'une part qu'il était possible d'immobiliser une α-amylase maltogénique sur un support poreux et, d'autre part, qu'une telle α-amylase maltogénique était capable d'hydrolyser des oligosaccharides de DP3 à DP7.

L'invention propose donc une α-amylase maltogénique immobilisée, caractérisée par le fait qu'elle est adsorbée sur des particules d'au moins un support poreux. Avantageusement, un tel support poreux est choisi dans le groupe constitué par les résines phénoliques, les résines acryliques et les résines polystyréniques.

L'invention propose encore un procédé d'immobilisation d'une α-amylase maltogénique selon l'invention, caractérisé par le fait qu'il comprend les étapes consistant à :
(a) mettre en solution une α-amylase maltogénique ;
(b) mettre en suspension des particules d'au moins un support poreux ;
(c) mettre en contact ladite solution avec ladite suspension à température ambiante en vue de l'obtention d'une α-amylase maltogénique immobilisée.

L'invention propose également un procédé de fabrication d'un sirop riche en maltose, comprenant les étapes successives consistant à :
(a) effectuer une liquéfaction d'un lait d'amidon ;
(b) effectuer une saccharification du lait d'amidon liquéfié en présence d'une β-amylase et d'au moins une enzyme débranchante choisie dans le groupe constitué par les pullulanases et les isoamylases ;
(c) mettre en contact le lait d'amidon liquéfié et saccharifié avec une α-amylase maltogénique immobilisée selon l'invention en vue de l'obtention d'un sirop riche en maltose.

L'invention propose aussi un procédé de fabrication d'un sirop riche en maltose, comprenant les étapes successives consistant à :
(a) effectuer une liquéfaction d'un lait d'amidon ;
(b) effectuer une saccharification du lait d'amidon liquéfié en présence d'une β-amylase et d'au moins une enzyme débranchante choisie dans le groupe constitué par les pullulanases et les isoamylases ;
(c) effectuer un tamisage moléculaire du lait d'amidon liquéfié et saccharifié de manière à recueillir une fraction enrichie en maltose et une fraction appauvrie en maltose ;
(d) mettre en contact ladite fraction enrichie en maltose avec une α-amylase maltogénique immobilisée selon l'invention en vue de l'obtention d'un sirop riche en maltose.

L'invention concerne donc en premier lieu une α-amylase maltogénique immobilisée. Au sens de la présente invention, on entend par « α-amylase maltogénique » une α-amylase capable d'hydrolyser des oligosaccharides (de DP3 à DP7) et essentiellement le maltotriose (trisaccharide), en maltose et glucose. Conformément à la présente invention, l'α-amylase maltogénique est avantageusement l'une de celles commercialisées par la société NOVO, sous les noms Maltogénase® 4000 L et NOVAMYL®.

La société Demanderesse recommande toutefois de procéder à une purification de l'α-amylase maltogénique commercialisée avant d'en réaliser l'immobilisation.

Il a été en effet noté que les préparations enzymatiques commerciales pouvaient être contaminées par des activités enzymatiques parasites, telles des activités protéases ou glucosidases.

Cette purification complémentaire permet en outre d'augmenter l'activité spécifique de l'α-amylase maltogénique ainsi préparée, ce qui augmente d'autant le potentiel de conversion du maltotriose par l'enzyme sous sa forme immobilisée. La purification de l'α-amylase maltogénique commerciale peut être effectuée par tout moyen connu de l'homme de métier.

Avantageusement, cette purification peut être réalisée par la suite d'étapes consistant à dialyser la préparation commerciale (en utilisant par exemple une membrane de dialyse fournie par DISLAB, de marque ROTH®, seuil de coupure entre 10 et 20.000 daltons), précipiter la solution commerciale ainsi dialysée au sulfate d'ammonium à 50%, traiter ladite solution enrichie en α-amylase maltogénique par gel filtration, dialyser (en utilisant une membrane du même type que celle mentionnée ci-dessus), puis la soumettre à un traitement en chromatographie d'échange d'ions de type CM-Sephadex C-50. Il est alors possible d'augmenter l'activité spécifique de ladite enzyme de 7 à 10 fois.

L'enzyme immobilisée conforme à la présente invention se présente sous la forme de particules d'au moins un support poreux choisi parmi les résines phénoliques, acryliques ou polystyréniques et, de préférence, les résines phénoliques, sur lequel est adsorbée l'enzyme.

Selon un mode de réalisation préférentiel de l'invention, les particules du support présentent un diamètre moyen de pores compris entre 200 et 800 Å et de préférence, compris entre 200 et 650 Å. Elles présentent par ailleurs un diamètre de particules compris entre 0,2 et 1,2 mm et, de préférence, compris entre 0,5 et 0,8 mm.

Avantageusement, le support mis en oeuvre dans la présente invention est par exemple, la résine XAD761 commercialisée par la société ROHM & HAAS.

Pour préparer l'α-amylase maltogénique immobilisée selon l'invention, on met en oeuvre le procédé décrit ci-après ou un procédé équivalent. Après mise en solution d'une α-amylase maltogénique purifiée ou non et mise en suspension des particules du support poreux, on met en contact ladite solution avec ladite suspension pendant au moins 15 mn et de préférence au moins 1 heure à une température comprise entre 15 et 80°C.

Cette mise en contact peut s'effectuer à température ambiante, de manière continue (avec par exemple recyclage de la solution de l'enzyme) ou discontinue.

Avantageusement, la mise en contact de l'α-amylase maltogénique et les particules du support poreux peut s'effectuer à une température comprise entre 20 et 70°C de préférence comprise entre 40 et 65°C. La Société Demanderesse a en effet constaté que la cinétique de fixation de l'α-amylase maltogénique sur lesdites particules était plus rapide, et la quantité d'enzymes fixées plus importante, la température élevée favorisant les interactions hydrophobes entre l'enzyme et son support.

L'α-amylase maltogénique ainsi immobilisée conformément à la présente invention présente d'excellentes performances pour la fabrication de sirops riche en maltose. C'est pourquoi la présente invention concerne également un procédé de fabrication d'un sirop riche en maltose mettant en oeuvre l'α-amylase maltogénique selon l'invention.

La première étape du procédé conforme à l'invention est en soi connue. Elle consiste à liquéfier un lait d'amidon dont l'origine botanique peut être quelconque : il peut provenir du blé, du maïs ou de la pomme de terre par exemple.

Ce lait d'amidon ou de fécule est additionné d'acide dans le cas d'une liquéfaction dite acide, ou d'une α-amylase dans le cas d'une liquéfaction enzymatique.

Dans le procédé conforme à l'invention, on préfère effectuer une hydrolyse ménagée du lait d'amidon de façon à obtenir un lait d'amidon liquéfié à faible taux de transformation. Ainsi, les conditions de température, de pH, de taux d'enzyme et de calcium, connues de l'homme du métier, sont déterminées de manière telle qu'elles permettent d'obtenir un DE (Dextrose Equivalent) inférieur à 10, de préférence inférieur à 6, et plus particulièrement inférieur à 4.De préférence, l'étape de liquéfaction est conduite en deux sous-étapes, la première consistant à chauffer, pendant quelques minutes et à une température comprise entre 105 et 108°C, le lait d'amidon en présence d'une α-amylase (type TERMAMYL^{R} 120L commercialisée par la société NOVO) et d'un activateur à base de calcium, la seconde consistant à chauffer le lait d'amidon ainsi traité à une température comprise entre 95 et 100°C pendant une à deux heures.

Une fois l'étape de liquéfaction terminée, dans les conditions de teneur en matières sèches, de pH, de taux d'enzyme et de calcium bien connues de l'homme du métier, on procède à l'inhibition de l'α-amylase. Cette inhibition de l'α-amylase peut se faire de préférence par voie thermique, en procédant en sortie de liquéfaction à un choc thermique de quelques secondes à une température supérieure ou égale à 130°C.

On effectue ensuite la saccharification du lait d'amidon liquéfié au moyen d'une β-amylase telle que celle commercialisée par la société GENENCOR sous la dénomination SPEZYME® BBA 1500.

Lors de cette étape, il convient d'associer à la β-amylase une enzyme hydrolysant spécifiquement les liaisons α-1,6 de l'amidon. Cet ajout d'une enzyme débranchante permet d'une part d'accélérer les réactions d'hydrolyse sans simultanément accélérer les réactions de réversion et, d'autre part, de réduire la quantité d'oligosaccharides hautement branchés résistant normalement à l'action des enzymes maltogéniques.

Selon l'invention, l'enzyme débranchante est choisie dans le groupe constitué par les pullulanases et les isoamylases. La pullulanase est, par exemple, celle commercialisée par la société ABM sous la dénomination PULLUZYME® 750L. L'isoamylase est, par exemple, celle commercialisée par la société HAYASHIBARA.

Avantageusement, le procédé conforme à l'invention est mis en oeuvre en présence d'isoamylase pour laquelle la société demanderesse a constaté qu'elle permettait d'obtenir un sirop de maltose présentant une teneur en maltose plus élevée qu'en utilisant une pullulanase.

Dans un mode de réalisation particulier de l'invention, l'étape de saccharification peut être conduite également totalement ou partiellement en présence d'α-amylase fongique en utilisant la SPEZYME® DBA 1500 (commercialisée par la société GENENCOR) en lieu et place de la SPEZYME® BBA 1500 (commercialisée par la même société).

En fin de saccharification, il est possible d'ajouter un peu d'α-amylase, ce qui améliore généralement les étapes subséquentes de filtration. Les quantités et les conditions d'action des différentes enzymes mises en oeuvre dans les étapes de liquéfaction et de saccharification du lait d'amidon sont généralement celles qui sont recommandées pour l'hydrolyse de l'amidon et sont bien connues de l'homme du métier.

On effectue la saccharification à la β-amylase associée à l'enzyme débranchante jusqu'à ce que l'hydrolysat de maltose contienne au moins 75 % en poids de maltose et, de préférence, environ 80 % en poids de maltose. Elle dure au moins 24 heures.

L'hydrolysat ainsi saccharifié est ensuite filtré sur filtre à précouche ou par microfiltration sur membranes, puis déminéralisé et concentré.

A ce stade du procédé conforme à l'invention, deux variantes peuvent être mises en oeuvre. Selon une première variante, on poursuit la saccharification en mettant en contact le lait d'amidon liquéfié et saccharifié avec une α-amylase maltogénique immobilisée conforme à l'invention. Cette mise en contact peut se faire, par exemple, par passage du lait d'amidon liquéfié et saccharifié sur des colonnes, lits ou autres enceintes garnies par l'α-amylase maltogénique conforme à l'invention. Le débit de passage est à adapter non seulement en fonction de la teneur en trisaccharides (par exemple entre 5 et 15 bv/h (bed volumes/hours) pour des teneurs de 13 à 5 %) mais aussi de la matière sèche (à 10 % de trisaccharides, entre 6 et 4 bv/h pour des matières sèches de 20 à 30 %). A la suite de cette étape, il est alors éventuellement possible d'effectuer sur le sirop ainsi obtenu un tamisage moléculaire permettant de l'enrichir en maltose.

Selon une seconde variante du procédé conforme à l'invention, on effectue tout d'abord un tamisage moléculaire du lait d'amidon liquéfié et saccharifié de manière à recueillir une fraction enrichie en maltose et une fraction appauvrie en maltose. A la suite de quoi, on met en contact la fraction enrichie en maltose avec une α-amylase maltogénique conforme à l'invention comme cela a été décrit ci-dessus.

L' étape de tamisage moléculaire mise en oeuvre dans l'une ou l'autre des deux variantes du procédé conforme à l'invention peut consister, par exemple, en une étape de séparation chromatographique ou en une étape de séparation sur membranes.

L'étape de fractionnement chromatographique est effectuée de manière connue en soi, de façon discontinue ou continue (lit mobile simulé), sur des adsorbants du type résines cationiques, ou sur des zéolithes fortement acides, chargées préférentiellement à l'aide d'ions alcalins ou alcalino-terreux tels que le calcium ou le magnésium mais plus préférentiellement à l'aide d'ions sodium.

En lieu et place de l'étape de séparation chromatographique, il est possible, dans le procédé conforme à l'invention, de mettre en oeuvre une étape de séparation par nanofiltration sur membranes. Des membranes de différents diamètres de pores sont fabriquées à partir de nombreux polymères et copolymères du type polysulfones, polyamides, polyacrylonitrates, polycarbonates, polyfuranes, etc.

Des exemples de l'utilisation de telles membranes sont décrits notamment dans les documents US-A-4.511.654, US-A-4.429.122 et WO-A-95/10627.

Grâce au procédé conforme à l'invention qui tire profit des bénéfices obtenus des étapes d'hydrolyse mettant en oeuvre l'α-amylase maltogénique selon l'invention, il est possible d'obtenir, avec des rendements supérieurs à 90 %, un hydrolysat d'amidon dont la teneur en maltose est supérieure à 95 %, et même supérieure à 98 % lorsqu'une isoamylase est mise en oeuvre dans les étapes d'hydrolyse.

A ce stade du procédé conforme à l'invention, il est éventuellement possible d'effectuer sur l'hydrolysat (ou sirop de maltose) une cristallisation du maltose ou une hydrogénation catalytique.

L'hydrogénation d'un tel hydrolysat s'effectue conformément aux règles de l'art qui conduisent par exemple à la production de sorbitol à partir du glucose.

On peut utiliser pour cette étape aussi bien des catalyseurs à base de ruthénium que des catalyseurs au nickel de RANEY. On préfère cependant utiliser des catalyseurs au nickel de RANEY qui sont moins onéreux.

Dans la pratique, on utilise de 1 à 10 % en poids de catalyseur par rapport à la matière sèche de l'hydrolysat soumis à l'hydrogénation. L'hydrogénation s'effectue de préférence sur un hydrolysat dont la matière sèche est comprise entre 15 et 50 %, dans la pratique voisine de 30 à 45 %, sous une pression d'hydrogène comprise entre 20 et 200 bars. Elle peut être effectuée de manière continue ou discontinue.

Lorsque l'on opère de manière discontinue, la pression d'hydrogène utilisée est généralement comprise entre 30 et 60 bars et la température à laquelle se déroule l'hydrogénation est comprise entre 100 et 150°C. On veille aussi à maintenir le pH du milieu d'hydrogénation par l'addition de soude ou de carbonate de soude par exemple, mais sans dépasser un pH de 9,0. Cette manière de faire permet d'éviter l'apparition de produits de cracking ou d'isomérisation.

On arrête la réaction lorsque la teneur du milieu réactionnel en sucres réducteurs est devenue inférieure à 1 %, de préférence encore inférieure à 0,5 % et plus particulièrement inférieure à 0,1 %.

Après refroidissement du milieu réactionnel, on élimine le catalyseur par filtration et on déminéralise le sirop de maltitol ainsi obtenu sur des résines cationiques et anioniques. A ce stade, les sirops contiennent au moins 93 % de maltitol.

Le sirop de maltitol obtenu à l'étape d'hydrogénation précédente peut alors subir une étape de cristallisation de manière à obtenir du maltitol cristallisé.

Selon un mode de réalisation préféré du procédé conforme à l'invention, on met en oeuvre sur le sirop de maltitol obtenu à l'étape d'hydrogénation précédente, la succession des étapes suivantes consistant à :
- concentrer le sirop de maltitol ;
- cristalliser et séparer les cristaux de maltitol formés ;
- effectuer sur les eaux-mères de cristallisation un tamisage moléculaire et, en particulier, un fractionnement chromatographique de manière à obtenir une fraction enrichie en maltitol et une fraction appauvrie en maltitol ;
- recycler la fraction enrichie en maltitol en amont de l'étape de cristallisation ;
- effectuer éventuellement sur la fraction appauvrie en maltitol, une hydrolyse acide et/ou une hydrolyse enzymatique au moyen par exemple d'une amyloglucosidase immobilisée ou non ;
- effectuer éventuellement une hydrogénation de ladite fraction appauvrie en maltitol et hydrolysée pour la transformer en un sirop de sorbitol.

D'autres caractéristiques et avantages de l'invention apparaîtront clairement à la lecture des exemples qui suivent. Ils ne sont toutefois donnés ici qu'à titre illustratif et non limitatif.

### EXEMPLE 1

Support : résine XAD761 commercialisée par la Société ROHM & HAAS.

Solution d'enzyme : Maltogénase® 4000 L, commercialisée par la Société NOVO, diluée au demi.

La solution est percolée en circuit fermé sur une colonne garnie de la résine préalablement réhydratée, à un débit de l'ordre de 3 à 4 bvh pendant une nuit, à température ambiante. Le bilan de l'activité est effectué en dosant l'activité enzymatique de la solution (selon la méthode NOVO AF 203/1 - GB) avant et après percolation : 4445 unités ont été fixées par ml de support, soit l'équivalent d'1 ml de Maltogénase® 4000 L.

### EXEMPLE 2

Support : résine XAD 761 commercialisée par la société ROHM & HAAS.

Solution d'enzyme : Maltogénase® 4000L, commercialisée par la société NOVO, diluée au 9/20.

La solution est percolée en circuit fermée sur une colonne garnie de la résine préalablement réhydratée, à un débit de 10 bvh/h, soit 1000 l/h, à une température de 60°C, pendant 6 heures. Le bilan de l'activité est effectué en dosant l'activité enzymatique de la solution (selon la méthode NOVO AF 203/1 -GB) avant et après percolation : 4000 unités ont été fixées par ml de support, soit l'équivalent d'1 ml de Maltogénase® 4000L, avec un rendement de 89% exprimé en nombre d'unités d'α-amylase maltogénique fixé sur le nombre total d'unités d'α-amylase maltogénique introduites.

### EXEMPLE 3

Un lait d'amidon, à une matière sèche de 31 %, est liquéfié de manière classique à l'aide de 0,2 % de TERMAMYL® 120L (α-amylase commercialisée par la société NOVO) à un pH de 5,7 à 6,5 jusqu'à un DE légèrement inférieur à 4.

On chauffe ensuite le milieu réactionnel pendant quelques secondes à 140°C de manière à inhiber l'α-amylase, puis on ajuste le pH entre 5 et 5,5 et la température à 55°C.

La saccharification est conduite à une matière sèche de 25 %, ou légèrement inférieure, en présence de pullulanase (PULLUZYME® 750L commercialisée par la société ABM) et de β-amylase (SPEZYME® BBA commercialisée par la société GENENCOR) à des doses respectives de 0,1 % et 0,05 % sur matière sèche.

La saccharification, qui dure environ 48 heures, donne un hydrolysat montrant la composition suivante, DP1 : 1,4 %, DP2 : 82,4 %, DP3 : 13,2 %, DP4 et plus : 2,6 %.

L'hydrolysat subit ensuite une purification classique par filtration, décoloration et déminéralisation puis est concentré à environ 20 % de matière sèche et ajusté à pH 5,5.

### EXEMPLE 4

On fait passer l'hydrolysat de l'exemple 3 sur une colonne garnie de l'enzyme obtenue à l'exemple 1 et thermostatée à 60°C. La composition de l'hydrolysat de maltose obtenu en fonction du débit est la suivante :

| Débit (bv/h) | DP1 | DP2 | DP3 | DP4 et + |
|---|---|---|---|---|
| 5 | 6,2 | 90,5 | 0,8 | 2,4 |
| 6 | 6,3 | 90,1 | 1 | 2,5 |

### EXEMPLE 5

On procède à une étape de chromatographie continue de l'hydrolysat de maltose tel qu'obtenu dans l'exemple 3 ci-dessus de la manière suivante.

Quatre colonnes d'un litre de résine PCR 732 sodique thermostatées à 75°C sont assemblées en série et alimentées en continu avec l'hydrolysat de maltose amené à une matière sèche de 60 % en poids, à un débit de 110 ml/h.

En sortie de colonne on récupère les fractions enrichies en maltose de composition suivante :
DP1 : 1,5 %, DP2 : 94 %, DP3 : 4,5 %.

Le rendement chromatographique en maltose est de 91,5 %.

De la même manière que dans l'exemple 3, on fait passer ces fractions enrichies en maltose sur une colonne garnie de l'enzyme obtenue à l'exemple 1 et thermostatée à 60°C. La composition du sirop de maltose obtenu en fonction du débit est la suivante :

| Débit (bv/h) | DP1 | DP2 | DP3 |
|---|---|---|---|
| 10 | 4 | 95,5 | 0,5 |

### EXEMPLE 6

Le sirop de maltose obtenu dans l'exemple 5 ci-dessus est soumis à une étape de cristallisation du maltose de la manière suivante. On prépare une solution de maltose d'une matière sèche de 75 % en poids à une température de 75°C. On ensemence la solution de maltose avec 5 % en poids de germes de cristaux de maltose et refroidit la solution de 75°C à 40°C, à raison de 0,5°C par heure, en agitant la solution à 50 tours/min dans un cristallisoir à double paroi.

En fin de cristallisation, les cristaux sont séparés de la liqueur mère à l'aide d'une essoreuse centrifuge conventionnelle.

Le rendement de cristallisation est de 50 % en poids exprimé en poids de maltose cristallisé sur le poids de maltose de départ. La pureté en maltose des cristaux récupérés est de 97,5 % sur sec. La teneur en eau est de 5 %.

### EXEMPLE 7

Le sirop de maltose issu de l'exemple 5 est déminéralisé puis hydrogéné dans les conditions suivantes :

| | |
|---|---|
| Matière sèche | 40 % |
| Température | 115°C |
| Dose de catalyseur | 5 % P/P sec |
| Pression d'H₂ | 50 bars |

On arrête la réaction quand les sucres réducteurs sont inférieurs à 0,3 %. Le milieu est alors filtré, déminéralisé et concentré à 85 % de matière sèche ; sa composition est :

| | |
|---|---|
| Sorbitol | 5,5 % |
| Maltitol | 94,0 % |
| Supérieurs hydrogénés | 0,5 % |

On procède alors à l'étape de cristallisation par refroidissement de 75 à 25°C, à raison de 0,5°C/heure sous agitation lente, avec amorçage de 6 % P/P sec de maltitol cristallisé de granulométrie comprise entre 200 et 250µm.

Après turbinage, les cristaux sont séchés et présentent une richesse de 99,7 % ; les eaux-mères sont ajustées à 60 % de matière sèche et chromatographiées.

Quatre colonnes d'un litre de résine PCR732, sous forme calcium, thermostatées à 85°C sont assemblées en série et alimentées de façon continue à un débit de 120 ml/h. Le rendement en maltitol est de 90,7 % et la fraction noble (fraction riche en maltitol) présente la composition suivante : sorbitol 4,5 % ; maltitol 95 % ; supérieurs hydrogénés : 0,5 %

La fraction appauvrie en maltitol, contenant 53,5 % de sorbitol, 42,5 % de maltitol et 4 % de supérieurs hydrogénés, est ensuite soumise à une étape d'hydrolyse acide.

L'hydrolyse de la fraction appauvrie est réalisée en continu, sur une résine échangeuse de cations, de type C145 de Purolite, sous forme H⁺ placée dans une colonne thermostatée à 115°C ; en alimentant la colonne à 1 bv/h avec la solution concentrée à 40 %, la composition suivante est obtenue : sorbitol : 70,5 % ; maltitol : 12,3 % ; supérieurs : 0,4 % ; glucose : 16,8 %.

Cette solution est ensuite déminéralisée et hydrogénée dans les conditions suivantes :

| | |
|---|---|
| Matière sèche | 40 % |
| Température | 135°C |
| Dose de catalyseur | 5 % P/Psec |
| Pression d'hydrogène | 50 bars, |

jusqu'à obtenir un taux de sucres réducteurs libres inférieur à 0,1 %.

## Revendications

1. α-amylase maltogénique immobilisée, caractérisée par le fait qu'elle est adsorbée sur des particules d'au moins un support poreux.

2. α-amylase maltogénique immobilisée selon la revendication 1, caractérisée par le fait que les particules du support présentent un diamètre moyen des pores compris entre 200 et 800 Å et un diamètre de particules compris entre 0,2 et 1,2 mm.

3. Procédé d'immobilisation d'une α-amylase maltogénique conforme à la revendication 1 ou 2, caractérisé par le fait qu'il comprend les étapes consistant à :
(a) mettre en solution une α-amylase maltogénique ;
(b) mettre en suspension des particules d'au moins un support poreux ;
(c) mettre en contact ladite solution avec ladite suspension pendant au moins 1 heure à une température comprise entre 15 et 80°C, de préférence entre 20 et 70°C et plus préférentiellement à une température comprise entre 40 et 65°C en vue de l'obtention d'une α-amylase maltogénique immobilisée.

4. Procédé de fabrication d'un sirop riche en maltose, comprenant les étapes successives consistant à :
(a) effectuer une liquéfaction d'un lait d'amidon ;
(b) effectuer une saccharification du lait d'amidon liquéfié en présence d'une β-amylase et d'au moins une enzyme débranchante choisie dans le groupe constitué par les pullulanases et les isoamylases ;
(c) mettre en contact le lait d'amidon liquéfié et saccharifié avec une α-amylase maltogénique immobilisée conforme à la revendication 1 ou 2 en vue de l'obtention d'un sirop riche en maltose.

5. Procédé selon la revendication 4, caractérisé par le fait que l'on effectue un tamisage moléculaire du sirop obtenu à l'étape (c).

6. Procédé de fabrication d'un sirop riche en maltose, comprenant les étapes successives consistant à :
(a) effectuer une liquéfaction d'un lait d'amidon ;
(b) effectuer une saccharification du lait d'amidon liquéfié en présence d'une β-amylase et d'au moins un enzyme débranchante choisie dans le groupe constitué par les pullulanases et les isoamylases ;
(c) effectuer un tamisage moléculaire du lait d'amidon liquéfié et saccharifié de manière à recueillir une fraction enrichie en maltose et une fraction appauvrie en maltose ;
(d) mettre en contact ladite fraction enrichie en maltose avec une α-amylase maltogénique immobilisée conforme à la revendication 1 et 2 en vue de l'obtention d'un sirop riche en maltose.

7. Procédé de fabrication de maltose cristallisé par cristallisation d'un sirop riche en maltose, caractérisé par le fait que le sirop riche en maltose est obtenu par la mise en oeuvre d'un procédé conforme à l'une quelconque des revendications 4 à 6.

8. Procédé de fabrication d'un sirop riche en maltitol par hydrogénation d'un sirop riche en maltose, caractérisé par le fait que le sirop riche en maltose est obtenu par la mise en oeuvre d'un procédé conforme à l'une quelconque des revendications 4 à 6.

9. Procédé de fabrication de maltitol cristallisé par cristallisation d'un sirop riche en maltitol, caractérisé par le fait que le sirop riche en maltitol est obtenu par la mise en oeuvre d'un procédé conforme à la revendication 8.

10. Procédé selon la revendication 9, caractérisé par le fait qu'il comprend les étapes consistant à :
(a) cristalliser le sirop en maltitol ;
(b) effectuer un tamisage moléculaire des eaux-mères de cristallisation de manière à recueillir une fraction enrichie en maltitol et une fraction appauvrie en maltitol ;
(c) recycler ladite fraction riche en maltitol en amont de l'étape de cristallisation ;
(d) effectuer une hydrolyse acide et/ou enzymatique de ladite fraction appauvrie en maltitol.
(e) Effectuer une hydrogénation de ladite fraction appauvrie en maltitol et hydrolysée.
